# EUROPEAN PATENT SPECIFICATION

(11) **EP 2 683 392 B1**
(45) Date of publication and mention of the grant of the patent: **26.04.2017**
(21) Application number: 12755446.7
(22) Date of filing: 09.03.2012
(51) Int. Cl.: A61K 36/54, A61P 1/04

(54) **CINNAMOMUM BURMANII EXTRACT, EXTRACTION PROCESS AND ITS USE AS PROTON PUMP DOWN-REGULATOR, ENZYME INHIBITOR, AND MUCOPROTECTOR**
CINNAMOMUM-BURMANII-EXTRAKT, EXTRAKTIONSVERFAHREN UND SEINE VERWENDUNG ALS PROTONENPUMPEN-ABWÄRTSREGLER, ENZYMHEMMER UND MUKOPROTECTOR
EXTRAIT DE CINNAMOMUM BURMANII, PROCÉDÉ D'EXTRACTION ET SON UTILISATION EN TANT QUE RÉGULATEUR À LA BAISSE DE POMPE À PROTON, INHIBITEUR D'ENZYME, ET MUCOPROTECTEUR

(30) Priority: 09.03.2011 ID 20110157
(43) Date of publication of application: 15.01.2014
(73) Proprietor: PT. Dexa Medica, Palembang, 30115 (ID)
(72) Inventor: ARIFIN, Poppy Firzani, 16342 Depok (ID); R. W., Deasy Diah Dwi, 40522 Cimahi (ID); YULIANI, Veronika, 12130 Jakarta Selatan (ID); SINAMBELA, James M., 17148 Bekasi (ID); TJANDRAWINATA, Raymond R., 12310 Jakarta Selatan (ID)
(74) Representative: Petraz, Gilberto Luigi
(86) International application number: PCT/IB2012/051104
(87) International publication number: WO 2012/120475

(56) References cited:
- WO-A1-2008/059310
- WO-A1-2010/023572
- JP-A- S5 965 018
- JP-A- S58 129 002
- JP-A- 2002 187 843
- SHAN, B. ET AL.: 'Antibacterial Properties and Major Bioactive Components of Cinnamon Stick (Cinnamomum burmannii): Activity against Foodborne Pathogenic Bacteria' JOURNAL OF AGRICULTURAL AND FOOD CHEMISTRY vol. 55, no. 14, 2007, pages 5484 - 5490, XP002514013
- CAO, H. ET AL.: 'Cinnamon extract regulates glucose transporter and insulin-signaling gene expression in mouse adipocytes' PHYTOMEDICINE vol. 17, no. 13, 2010, pages 1027 - 1032, XP027417664
- TANAKA, S. ET AL.: 'Antiulcerogenic Compounds Isolated from Chinese Cinnamon' PLANTA MEDICA vol. 55, no. 3, 1989, pages 245 - 248, XP008170478

## Description

### Field of the invention

The present invention relates to the extract from *Cinnamomum burmannii* plant, which for the purpose of this invention is referred to as DLBS2411, and includes the extraction method as well as the biological activities of said extract as antiulcer agent, which include down-regulating proton pump H⁺/K⁺ ATPase, inhibiting enzyme, and conferring mucoprotection. Treatment with DLBS2411 is able to reduce the expression of mRNA H⁺/K⁺ ATPase in HEK 293 and gastric parietal cells. In addition, DLBS2411 inhibits the activity of gastric enzyme H⁺/K⁺ ATPase wherein the inhibition is affected by pH. Besides, DLBS2411 also has antioxidant activity which is shown by the increase of reducing power along with the increase of concentration given.

### Background of the invention

Gastric disease is a high prevalence disease in the world which includes dyspepsia, gastric ulcer, duodenal ulcer, gastroesophageal reflux disease (GERD), laryngopharyngeal reflux (LPR), Zollinger-Ellison syndromes, and also diseases which are triggered by the long-term use of medicine. Generally, this is caused by the increased activity of proton pump that promotes the excessive production of gastric acid and lead to gastric irritation. Enzyme that works during the excretion of gastric acid at the proton pump is known as H⁺/K⁺ ATPase enzyme. This enzyme is bound to the apical membrane in parietal cells. Inhibition of proton pump in the parietal cells that produce acid is deemed as an effective approach to reduce gastric acid, shown by the reduction of gastric acid secretion at the mRNA expression level and H⁺/K⁺ ATPase enzyme activity. H⁺/K^{T} ATPase genes are not only expressed in gastric parietal cells but also in liver and intestinal cells.

Currently, there are several medicines available in the market that works by inhibiting proton pump, such as omeprazole, lansoprazole, rabeprazole, and esomeprazole. However, the finding of new medicine to inhibit this proton pump with low adverse events is still needed and one possibility is coming from natural materials, such as *Cinnamomum burmannii*.

Document JP S59 65018 A discloses a treating and preventing agent for peptic ulcer, obtained by distributing and fractionating an extract of a cinnamon with hot water with ethyl ether, and recovering a water-soluble fraction to give a water-soluble constituent, and using the resultant water-soluble constituent derived from the cinnamon as an active constituent.

In this present disclosure the effect of DLBS2411, *Cinnamomum burmannii* extract, was investigated as antiulcer including its activity in down-regulating proton pump H⁺/K⁺ ATPase and as enzyme inhibitor, which are shown by the decrease in H⁺/K⁺ ATPase gene expression and activity of this enzyme. Prior to the present disclosure there has not been any study that explains DLBS2411, *Cinnamomum burmannii* extract as claimed in this invention. *burmannii*

### Brief description of the invention

The objects and/or solutions offered by the present disclosure will be set forth in the preferred embodiments. The embodiments illustrated serve the purpose of understanding the present disclosure without limiting the possibilities of other embodiments in variation and/or combination and/or modification, which can be learned from the practice of the present disclosure. The objects and/or solutions which are taught in the present disclosure will be realized from the elements and combination detailed in the claims herein.

To attain the solutions and in accordance with the objects of the present disclosure as explained in the embodiments and broadly described in this application, the first aspect of the present invention is directed to the extract of the plant in the genus Cinnamomum, preferably *Cinnamomum burmannii*, or its fraction(s) or its group of compound, a single active ingredient or in combination, in an effective amount or dosage to serve as antiulcer.

The present disclosure is also directed to the extract of the plant in the genus Cinnamomum, preferably *Cinnamomum burmannii*, or its fraction(s) or its group of compound, as a single active ingredient or in combination, in an effective amount or dosage which functions as down-regulator of H⁺/K⁺ ATPase, enzyme inhibitor and mucoprotector.

A further aspect of the present disclosure is directed to a pharmaceutical composition which comprises (1) extract of the plant in the genus Cinnamomum, preferably *Cinnamomum burmannii*, or its fraction (s) or its group of compound, in an effective amount or dosage, as a single active ingredient or in combination, and (2) carrier, excipients or additives that are pharmaceutically acceptable and physiologically suitable, which functions as antiulcer.

A still further aspect of the present disclosure is directed to the pharmaceutical composition or preparation which comprises (1) extract of the plant in the genus Cinnamomum, preferably *Cinnamomum burmannii,* or its fraction or its group of compound, in an effective dosage, as a single active ingredient or in combination, and (2) carrier, excipients or additives that are pharmaceutically acceptable and physiologically suitable, whereby the pharmaceutical composition or preparation is produced as solid, semi-solid, or liquid, in either sterile or non sterile form, which functions as antiulcer.

A further aspect of the present disclosure is also directed to food and beverage products which promotes health, which comprises extract of *Cinnamomum burmannii* or its fraction (s) or its group of compound, in an effective dosage, as a single active ingredient or in combination, which functions as antiulcer.

### Brief description of the drawings

The following drawings, which are incorporated in and constitute a part of the specification of the present application, illustrate one or several embodiments of the invention. The following drawings serve to explain the principles which are taught by the present invention.
Figure No. 1 is a diagram that shows the effect of DLBS2411 on the reduction of H⁺/K⁺ ATPase gene expression in HEK 293 cells which was illustrated by the result of Real Time PCR.
Figure No. 2 is a diagram that shows the effect of DLBS2411 on the reduction of H⁺/K⁺ ATPase gene expression in gastric parietal cells which was illustrated by the result of Real Time PCR.
Figure No. 3 is a diagram that shows the effect of DLBS2411 on the reduction of H⁺/K⁺ ATPase enzyme activity in various pH.
Figure No. 4 and 5 are diagrams that show the effect of treatment with and without DLBS2411 on H⁺/K⁺ ATPase enzyme kinetic activity.
Figure No. 6 is a diagram that shows the number of ptechiae on Wistar rats after administration of ethanol and DLBS2411.
Figure No. 7 is a diagram that shows the number of ptechiae on Wistar rats after administration of indomethacin and DLBS2411.
Figure No. 8 is a diagram that shows the effect of DLBS2411 on reduction potential.
Figure No. 9 is a diagram that shows the effect of DLBS2411 on the reduction of H⁺/K⁺ ATPase enzyme activity on Wistar rats which were previously treated with ethanol 15%.
Figure No. 10 is a diagram that shows the effect of DLBS2411 on the reduction of H⁺/K⁺ ATPase enzyme activity on Wistar rats which were previously treated with indomethacin 30 mg/kg.

### Detailed description of the invention

The present invention will be discussed in detail by giving examples without limiting the scope of the invention to the example provided.

Extract and/or fraction according to the teaching of the present invention is obtained from the bark of *Cinnamomum burmannii*. This plant grows in various areas, in and outside of Indonesia, which preferably is the one that grows in Padang, West Sumatra. It will be described herewith the extraction process and the effect of DLBS2411 as down-regulator of H⁺/K⁺ ATPase proton pump and enzyme inhibitor which is further applied as antiulcer and mucoprotector investigated in vitro, in vivo, and ex vivo, analyzed using Real Time PCR; and enzyme activity assay.

### A. Extraction process

Extraction process is begun with grinding the raw material of *Cinnamomum burmannii*, preferably the bark, until homogenous. The homogenous material is put into the extractor containing hot water with temperature of 50-100°C. The mixture is extracted for 1-2 hours. Subsequently, the mixture is filtered to remove the miscella and the resulting filtrate is evaporated by vacuum method using rotary evaporator (rotavapor) to produce concentrate.

The concentrate is fractionated by liquid - liquid extraction using organic solvent(s) to separate the unwanted organic component(s) and the water phase was collected. Organic solvent(s) which can be used in this process includes but not limited to chloroform, dichloromethane, hexane, ethyl acetate and butanol in absolute condition with certain ratio to the concentrate. The preferred ratio is 1:1-2:1. The water phase is collected by separating it from the organic phase. Extraction is done several times, preferably 1-10 times, to optimize the collection of water phase.

Vacuum evaporation is done to produce concentrate which will be further dried using dryer, and finally dry fraction is obtained.

Dry fraction is then milled or crushed until it becomes fine powder, and then it is filtered using shifter with appropriate mesh number. This dry fraction will be what is subsequently called DLBS2411.

### Example 1: Extraction to obtain concentrate

According to the detailed description of the present invention, the extraction process in small scale can be done by soaking 10 gram of *Cinnamomum burmannii* bark in hot water with temperature of 70°C with ratio 10 times of the weight of the raw material. The resulting miscella is evaporated using rotavapor which yields 10 ml of concentrate.

### Example 2: Fractionation to obtain dry water fraction

According to the detailed description of the present invention, fractionation process in small scale can be done using 10 ml of concentrate. Liquid-liquid extraction is done by using several organic solvents (for example n-butanol, n-hexane, ethyl acetate and chloroform) in 1 time the volume of the concentrate. Liquid-liquid extraction is done for 3 times. Water fraction is collected and evaporated using rotavapor. The yield obtained in this process is dry fraction in the amount of 80 mg.

### B. DLBS2411 as down-regulator of H⁺/K⁺ ATPase gene expression

H⁺/K⁺ ATPase gene is the gene that plays a role in the proton pump process. This gene is expressed in HEK 293 and gastric parietal cells which regulates acid secretion.

Firstly, kidney HEK 293 and gastric parietal cells were isolated from Wistar rats. Kidney HEK 293 cells were cultured in MEM medium and gastric parietal cells in RPMI medium supplemented with 10% serum and 1% antibiotic Penicilin/Streptomycin in 6-well plate. The medium were incubated at 37°C, 5% CO₂ for 24 hours. Both of the medium were then changed to medium without serum, and incubated for 18-24 hours before treatment was applied. HEK 293 and gastric parietal cells contained in well plate were treated with DLBS2411 in various concentrations: 10 µg/mL; 25 µg/mL; and 50 µg/mL. Next, each cell in medium without serum that had been treated with DLBS2411 was incubated for 24 hours.

### RNA isolation and Real Time-PCR

In order to identify the reduction of H⁺/K⁺ ATPase mRNA expression in cell after administration of DLBS2411, Real Time PCR assay was done. Total RNA was isolated from HEK 293 and gastric parietal cells. The product of RNA isolation was quantified using spectrophotometer at wavelength of 260 nm and 280 nm, and visualized by electrophoresis on agarose gel. RNA was then used for Real Time PCR process whereby specific primer was used. Real Time PCR was done using PCR instrument at the optimized condition, and using primer which is specific for H⁺/K⁺ ATPase with DNA sequence is as follows:
Forward: 5' GCT GCA GCT CCA TCC TTA TC 3'
Reverse: 5' AGG CGG GTA GTC CTT CTC AT 3'

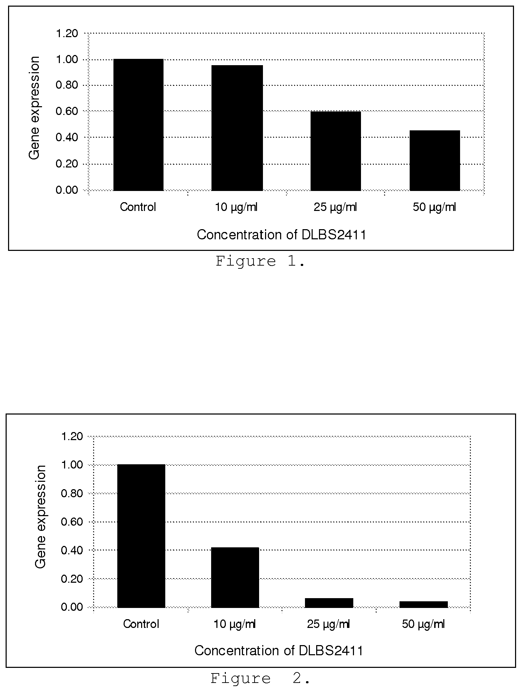

From the result of PCR assay using primer of H⁺/K⁺ ATPase quantitatively, the decrease of H⁺/K⁺ ATPase mRNA expression level was shown in HEK 293 and gastric parietal cells after administration of DLBS2411 (Figure 1 and 2). The result also reveals that DLBS2411 works better in HEK 293 cells compared to gastric parietal cells, shown by the ability of the extract in various dosages to decrease the H⁺/K⁺ ATPase gene expression. The gene expression declined as the dosage of DLBS2411 increased.

### C. DLBS2411 as the inhibitor of H⁺/K⁺ ATPase enzyme activity

There are several factors that influence enzyme activity, including temperature, pH and inhibitor. In this present invention, the effect of DLBS2411 as inhibitor was observed on H⁺/K⁺ ATPase enzyme activity wherein the assay was based on the assessment of the anorganic phosphate released from hydrolisis of ATP. This assay was done using Enzcheck Phosphate Assay Kit (Molecular Probes) according to the manual available from the kit. This enzyme assay was done in gastric parietal cells which are isolated from Wistar rats and cultured with addition of 30 µg/mL DLBS2411 wherein the pH levels during the assay were varied as follows: 7,4; 4; and 2. Besides, H⁺/K⁺ ATPase enzyme kinetic assay in gastric parietal cells was done with and without treatment of DLBS2411.

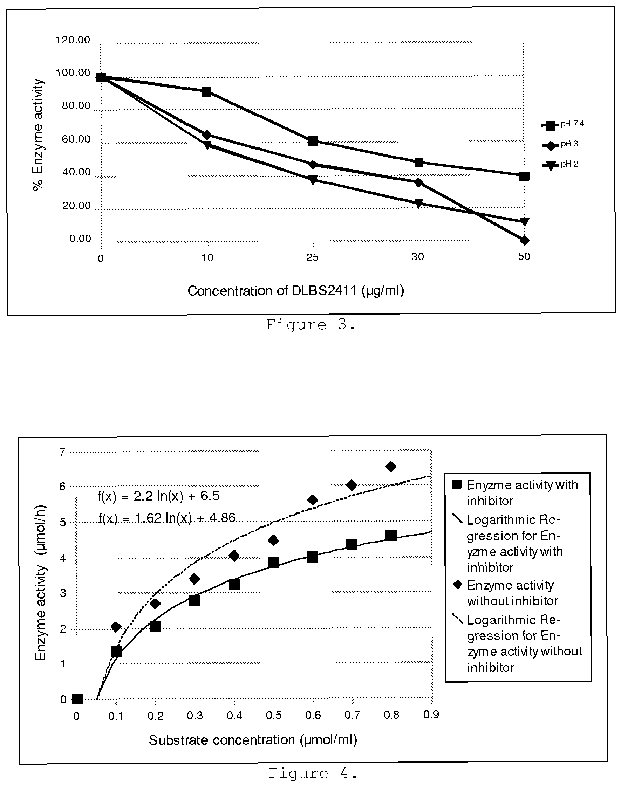

From the result of H⁺/K⁺ ATPase enzyme activity in gastric parietal cells, it was obtained that DLBS2411 had the ability to decrease the enzyme activity as the dosage of DLBS2411 increased (Figure 3). The decrease of activity was also influenced by pH. This condition was seen by the decrease of IC₅₀ value wherein at pH 7.4, IC₅₀ was 29.20 µg/ml; pH 4 was 18.70 µg/ml and pH 2 IC₅₀ was 9.20 µg/ml. The data showed that DLBS2411 was able to work effectively in inhibiting proton pump activity in the stomach, particularly in the condition of low gastric pH. The reduction of H⁺/K⁺ ATPase enzyme activity is in line with that of its gene expression which had been previously tested. This data indicates that the inhibition of enzyme activity is related to the decrease of the gene transcription after being treated with DLBS2411.

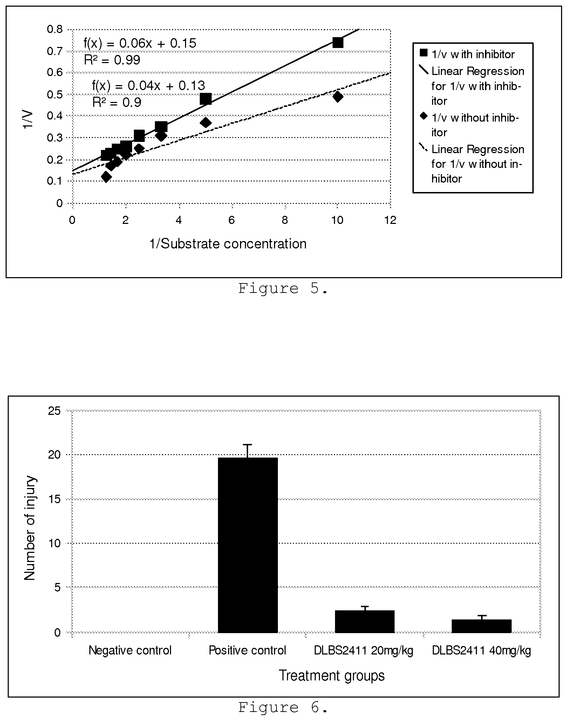

Moreover, from the enzyme kinetic test, it was found that the test without DLBS2411 treatment resulted in Km (Michaelis constant) value as much as 0.3075 µmmol/ml and Vmax as much as 7.69 µmmolPi/h, while with DLBS2411, the Km value was 0.4002 µmmol/ml and Vmax was 6.67 µmmolPi/h (Figure 4 and 5). The Km value is used to measure enzyme-substrate affinity. This is related to the equilibrium dissociation constant of the E-S complex. It is also known in the art that small Km value means that E-S complex is in best condition and the affinity of enzyme to substrate is high, and vice versa. In Figure 5, it is shown that DLBS2411 has a characteristic as inhibitor of enzyme activity by way of competitive inhibition. This competitive inhibitor is bound to the active site of the enzyme which prevents the enzyme to bind to substrate. The result obtained from the enzyme kinetic assay according to the teaching of the present invention reflects the characteristics of DLBS2411 as competitive inhibitor whereby, DLBS2411 inhibits H⁺/K⁺ ATPase enzyme activity.

### D. In vivo assay of ulceration-inhibitory activity of DLBS2411

In this present disclosure DLBS2411 activity in inhibiting the ulceration was investigated by in vivo test using Wistar rats.

Test animals that were used in this experiment were Wistar rats (*Rattus norvegicus* Wistar strain), male with the age of 2 months and weight of 200-210 g. The animals were housed individually with access for food and drink ad libitum. They were maintained at the temperature of 22-23°C with a 12-hour light/dark cycle according to the guidelines of use and care of test animal which is recognized by Association for Assessment and Accreditation of Laboratory Animal Care (AAALAC).

Test animals were allocated into two main groups: first, test animals receiving induction of gastric ulcer by treatment with ethanol; second, test animals receiving induction of gastric ulcer by treatment with indomethacin. The animals experienced gastric injury which characterized the beginning of ulceration. In this research, the occurence of gastric injury was observed by the formation of ptechiae, marked by red spots on the rat's stomach in which previously induced by ethanol and indomethacin.

### 1. Gastric ulcer induction using ethanol in Wistar rats

Ethanol-induced ulceration has been widely used in investigating the effect of plant extract as mucoprotector. The ethanol induced gastric injury is associated with oxidative stress, increase of lipid peroxidation and generation of free radical. In this present invention, potency of DLBS2411 as mucoprotector is shown by its activity in inhibiting gastric injury.

Test animals were allocated into 4 groups: (1) negative control group, (2) positive control group, (3) treated group with DLBS2411 20 mg/kg bw, (4) treated group with 40 mg/kg bw. Negative control group was the group wherein the test animal only received treatment with normal saline. Other groups received early treatment with ethanol 80% and then induced with ethanol 15% for one week. After one week, positive control group was treated with normal saline, while 2 treated groups were induced with DLBS2411 in 2 different dosages: 20 and 40 mg/kg bw. At the end of treatment period, the test animals were fasted overnight. Euthanasia was done to the animals by decapitation, and then the gaster of animals was observed to check the development of ulcer.

By this treatment, it was found that ulceration was developed in Wistar rats in the form of ptechiae as much as 19-67±1.53 red spots in the positive control group whereby the test animals was given treatment with normal saline solution (Figure 6). On the other hand, in the test animals which received DLBS2411 treatment, the decrease in the number of gastric injury was found wherein the test animals receiving 20 mg/kg bw of DLBS2411 had 2.33±0.58 spots of ptechiae and that of receiving 40 mg/kg bw had 1.33±0.58 spots of ptechiae. This condition showed that the development of ulcer observed by the formation of ptechiae can be suppressed with the administration of DLBS2411 in a dose-dependent manner.

### 2. Gastric ulcer induction using indomethacin in Wistar rats

Indomethacin-induced ulceration has also been widely used in investigating the effect of plant extract as mucoprotector. Mechanism of indomethacin-induced ulceration includes inhibition of prostaglandin biosynthesis, reduction of gastric mucosal blood flow and disorder in tissue healing. In the present invention, the potency of DLBS2411 as mucoprotector is shown by its activity in inhibiting gastric injury.

Test animals were allocated into 4 groups: (1) negative control group, (2) positive control group, (3), treated group with DLBS2411 20 mg/kg bw, (4) treated group with 40 mg/kg bw. Negative control group was the group wherein the test animal only received treatment with normal saline. Positive control groups received normal saline and indomethacin, first and second treated groups wherein the test animal received treatment with DLBS2411 20 and 40 mg/kg bw, respectively, for one week, and then treated with indomethacin 30 mg/kg bw. Induction of indomethacin was done after test animals fasted overnight; surgery was done afterwards. Test animals were anesthetized prior to midline laparotomy incision. Pylorus was identified and then it was bound using silk yarn with the size of 3/0. Subsequently, test animal received indomethacin 30 mg/kg bw and water. After treatment for 6 hours, euthanasia was done to the animals by decapitation, and then the gastric of animals was observed to check the development of ulcer.

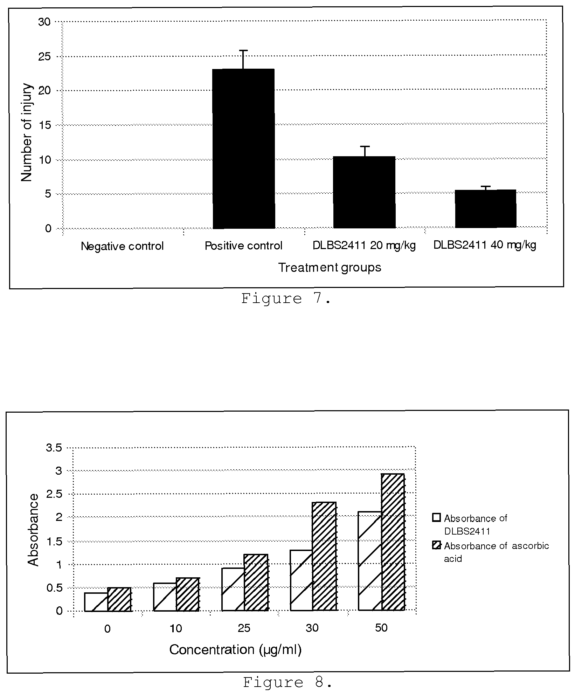

Ulceration in the test animals receiving 30 mg/kg bw of indomethacin was observed by 23±2.65 of ptechiae (Figure 7). The number of the ptechiae decreased in the group treated with DLBS2411 20 and 40 mg/kg, wherein the number became 10.33±1.53 and 5.33±0.58 ptechiae, respectively. Those data shows that DLBS2411 had an effect in ulceration inhibition, shown by the decrease in the number of gastric injury of Wistar rats. Such condition reveals the activity of DLBS2411 as mucoprotector. Induction of DLBS2411 to Wistar rats did not cause significant side effects. Moreover, it was also found that it did not cause abnormality to the kidney, heart, liver, spleen, intestine and lung. Those data was also supported by the toxicity study which shows the normal activity of rats. In addition, observation using microscope also showed normal condition of the internal organs of rats.

### E. Activity of DLBS2411 as antioxidant

Reducing power assay was done using Oyaizu method (1986). DLBS2411 (0-50 µg/mL) was mixed with phosphate buffer and potassium ferricyanide. The mixture was incubated at 50°C for 20 minutes and then it was added with TCA solution and centrifuged at 3000 rpm for 10 minutes. Upper layer was collected and mixed with distilled water and ferric chloride solution. Absorbance was measured using spectrophotometer at the wavelength of 700 nm. Absorbance value obtained from this assay shows the reducing power of extract. Ascorbic acid (vitamin C) was used as control.

The result showed that DLBS2411 had a reducing power which increased as the dosage of DLBS2411 increased (Figure 8). Ulceration is mostly happened due to hyperoxidation process which can lead to stomach ulcer. With high reducing power, it was expected that this extract was able to reduce the hyperoxidation process. This reducing power showed that DLBS2411 has potential activity as antioxidant.

### F. Ex vivo assay of H⁺/K⁺ ATPase enzyme activity

Enzyme assay was done in gastric parietal cells isolated from Wistar rats from 2 different treated groups which were the group previously treated with ethanol 15% and other group with indomethacin 30 mg/kg. Both treated groups were given DLBS2411 20 mg/kg bw and 40 mg/kg bw.

Enzyme activity assay was based on the quantification of inorganic phosphate released from hydrolysis of ATP. This assay was done using Enzcheck Phosphate Assay Kit (Molecular Probes) according to the manual available from the kit.

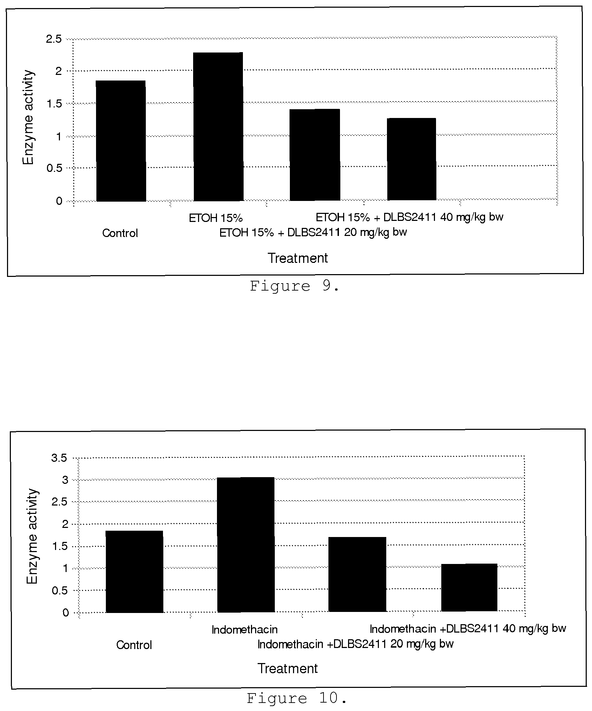

From the result of H⁺/K⁺ ATPase enzyme activity assay in gastric parietal cells of rats, it was found that enzyme activity assay increased with addition of ethanol 15% (Figure 9) and indomethacin (Figure 10) whereby indicating the hyperacidity which caused ulceration. However, the enzyme activity decreased after treatment with DLBS2411. This might happen due to the interaction of compounds contained in the extract with H⁺/K⁺ ATPase enzyme which inhibited the activity of the enzyme. The decrease of enzyme activity indicates that DLBS2411 is able to suppress hyperacidity and reduce the chance of ulceration.

### G. Compositions, Pharmaceutical Preparations, and Nutraceuticals

The present disclosure includes the composition and pharmaceutical preparation that contains DLBS2411 in an effective amount or dosage as an active ingredient in both single and in combination, including carrier, excipients or additives that are pharmaceutically acceptable and physiologically suitable.

In the process of preparing pharmaceutical composition as taught in the present invention, active ingredients DLBS2411 can be mixed with, or dissolved in excipient(s), or contained in carrier that can be made in the form of capsule, sachets, paper, as well as other packaging materials.

If pharmaceutically acceptable excipient is used as a solvent, the excipient can be in form of solid, semi-solid or liquid (oral and injection), that reacts as a carrier or medium for the active ingredient. Thus, pharmaceutical composition according to this invention can be made in form of pill, capsule, tablet, powder, sachet, solution, syrup, emulsion, suspension, effervescence tablets, gel, ointment, cream, and mouthwash, massage oil, suppository, or injection. Beside that, pharmaceutical composition comprising DLBS2411 according to this invention can also be made as supplement, vitamin, also food and beverage production.

Some examples of suitable excipients are microcrystalline cellulose, gelatin, lactose, dextrose, sucrose, sorbitol, mannitol, starch, calcium phosphate, calcium silicate, and others. Formulations that are appropriate with the teachings of the present invention may also contain lubricant (such as: talc, stearic magnesium, mineral oil), wetting agent(s), preservatives, sweeteners and flavoring.

Composition according to the present invention can be made with formulation that causes the active ingredient to be released directly, sustained or controlled after the patient receives such dosage forms using methods that have been applied in pharmaceutical industry. Tablet or pill according to the present invention can be layered to extend the half-life of the extract thus its frequency of use can be reduced.

Method of formulating this composition in a solid form, such as tablet, can be done by mixing the active ingredient of the extract and/or fraction of DLBS2411, with excipient(s) to form an initial formulation containing homogenous mixture from the composition according to the present invention. The initial formulation is a mixture containing the active ingredient of the DLBS2411 dispersed homogeneously, so it can be distributed according to the proper dosage into forms such as, for example, capsule, tablet, or pill.

Tablet or pill, according to the present invention can be given an additional protection layer to reduce or cover the bitter taste from the composition or the active substance DLBS2411. Similarly, for the semi-solid and liquid forms, according to the present invention, additional substances can be given to reduce bitterness or smell, if desired, from the composition or active substance DLBS2411.

DLBS2411 in an effective amount or dosage according to the present invention is the dosage of extract that can inhibit proton pump. An effective amount depends on the physical condition of the patient (including weight, age, and other factors; includes on type, size and number of cells and other targeted pathologics condition).

### H. Industrial application

Extract, and/or pharmaceutical composition from DLBS2411, can be used in industrial scale in the production of extract, dry powder extract, and/or pharmaceutical composition for oral dosage that can either be solid, semi-solid or liquid, sterile or non-sterile in its use as antiulcer by down-regulating H⁺/K⁺ ATPase proton pump, inhibiting enzyme and conferring mucoprotection.

## Claims

1. An extract comprising *Cinnamomum burmannii*, and/or its fraction or its group of compound derived therefrom, for use in the treatment of ulcer, in pharmaceutical preparation, as a single active ingredient or in combination, in an amount or dose that is effective as antiulcer.

2. Extract and/or fraction for use according to claim 1, whereby said extract and/or fraction has the activity as down-regulator of H⁺/K⁺ ATPase proton pump.

3. Extract and/or fraction for use according to claim 1, whereby said extract and/or fraction has the activity as inhibitor of H⁺/K⁺ ATPase enzyme.

4. Extract and/or fraction for use according to claim 1, whereby said extract and/or fraction has the activity as mucoprotector.

5. Extract and/or fraction for use according to claim 1, whereby such extract is obtained by the process comprising the steps of:
(a) extraction using hot water at temperature of 50-100°C,
(b) liquid-liquid extraction using organic solvent to obtain water fraction.

6. Extract and/or fraction for use according to claim 5, whereby the process further comprises the step of repetition of step (b) to optimize the collection of water fraction.

7. Extract and/or fraction for use according to claim 5, whereby the organic solvent(s) at step (b) is selected from the group consisting of chloroform, dichloromethane, hexane, ethyl acetate and butanol.

8. A pharmaceutical composition for use in the treatment of ulcer comprising the extract and/or fraction according to claim 1, as a single active ingredient or in combination, and carrier, excipient(s) or additive(s) that are pharmaceutically acceptable and physiologically suitable.

9. A pharmaceutical composition for use according to claim 8, wherein the pharmaceutical composition is made in the form of solid, semi-solid, or liquid, in sterile or non-sterile product(s).

10. A food or beverage product directed to promote health benefit comprising said extract and/or fraction according to claim 1, as a single active ingredient or in combination for use in the treatment of ulcer.

## Patentansprüche

1. Ein Auszug umfassend *Cinnamonum burmannii* und/oder seine Fraktionen oder seine Gruppe von daraus abgeleiteter Verbindung, zur Verwendung in der Behandlung eines Ulcus, in einer pharmazeutischen Zubereitung, als ein einzelner aktiver Inhaltsstoff oder in Kombination, in einer Menge oder Dosis, die als Anti-Ulcus-Mittel wirksam ist.

2. Auszug und/oder Fraktion zur Verwendung gemäß Anspruch 1, wobei der Auszug und/oder die Fraktion Wirksamkeit als Runterregulierer der H⁺/K⁺-ATPase-Protonenpumpe hat.

3. Auszug und/oder Fraktion zur Verwendung gemäß Anspruch 1, wobei der Auszug und/oder die Fraktion Wirksamkeit als Inhibitor des H⁺/K⁺-ATPase-Enzyms hat.

4. Auszug und/oder Fraktion zur Verwendung gemäß Anspruch 1, wobei der Auszug und/oder die Fraktion Wirksamkeit als Schleimhaut-Schutz hat.

5. Auszug und/oder Fraktion zur Verwendung gemäß Anspruch 1, wobei solch ein Auszug durch das Verfahren umfassend die Schritte des
(a) Extrahierens unter Verwendung von heißen Wasser bei einer Temperatur von 50-100 °C,
(b) Flüssig-Flüssig-Extrahierens unter Verwendung organischen Lösemittels, um eine Wasser-Fraktion zu erhalten
erhalten wird.

6. Auszug und/oder Fraktion zur Verwendung gemäß Anspruch 5, wobei das Verfahren des Weiteren den Schritt des Wiederholens von Schritt (b) umfasst, um das Sammeln der Wasser-Fraktion zu optimieren.

7. Auszug und/oder Fraktion zur Verwendung gemäß Anspruch 5, wobei das/die organische(n) Lösemittel in Schritt (b) ausgewählt ist aus der Gruppe bestehend aus Chloroform, Dichlormethan, Hexan, Ethylacetat und Butanol.

8. Eine pharmazeutische Zusammensetzung zur Verwendung in der Behandlung eines Ulcus umfassend den Auszug und/oder die Fraktion gemäß Anspruch 1, als ein einzelner aktiver Inhaltsstoff oder in Kombination, und Träger, Arzneiträgerstoff(e) oder Additiv(e), die pharmazeutisch annehmbar und physiologisch geeignet sind.

9. Eine pharmazeutische Zusammensetzung zur Verwendung gemäß Anspruch 8, wobei die pharmazeutische Zusammensetzung in Form eines Feststoffs, Halbfeststoffs oder einer Flüssigkeit, in sterilen oder nicht-sterilen Produkt(en) gefertigt ist.

10. Ein Lebensmittel- oder Getränkeprodukt bestimmt zur Förderung von Gesundheitsvorteilen, umfassend den Auszug und/oder die Fraktion gemäß Anspruch 1, als ein einzelner aktiver Inhaltsstoff oder in Kombination zur Verwendung in der Behandlung eines Ulcus.

## Revendications

1. Extrait comprenant Cinnamomum burmannii et/ou fraction de celui-ci ou groupe de composés dérivant de celui-ci, utilisables pour le traitement de l'ulcère, dans une préparation pharmaceutique, comme principe actif unique ou en combinaison, dans une quantité ou une dose efficace en tant qu'anti-ulcère.

2. Extrait et/ou fraction utilisable selon la revendication 1, ledit extrait et/ou ladite fraction ayant une activité de régulation à la baisse de la pompe à proton H⁺/K⁺ ATPase.

3. Extrait et/ou fraction utilisables selon la revendication 1, ledit extrait et/ou ladite fraction ayant une activité d'inhibition de l'enzyme H⁺/K⁺ ATPase.

4. Extrait et/ou fraction utilisables selon la revendication 1, ledit extrait et/ou ladite fraction ayant une activité de mucoprotection.

5. Extrait et/ou fraction utilisables selon la revendication 1, ledit extrait étant obtenu par le procédé comprenant les étapes suivantes :
(a) extraction à l'eau chaude à une température de 50 - 100°C,
(b) extraction liquide-liquide à l'aide d'un solvant organique pour obtenir la fraction d'eau.

6. Extrait et/ou fraction utilisables selon la revendication 5, le procédé comprenant par ailleurs l'étape de répétition de l'étape (b) pour optimiser la collection de la fraction d'eau.

7. Extrait et/ou fraction utilisables selon la revendication 5, le(s) solvant(s) organique(s) à l'étape (b) est(sont) choisi(s) dans le groupe constitué par chloroforme, dichlorométhane, héxane, éthyl acétate et butanol.

8. Composition pharmaceutique utilisable dans le traitement de l'ulcère comprenant l'extrait et/ou a fraction selon la revendication 1, comme principe actif unique ou en combinaison, et un vecteur, excipient(s) ou additif(s) pharmaceutiquement acceptables, et physiologiquement appropriés.

9. Composition pharmaceutique utilisable selon la revendication 8, dans laquelle la composition pharmaceutique est en forme de solide, semi-solide, ou liquide, dans un ou plusieurs produits stériles ou non-stériles.

10. Produit alimentaire ou boisson destiné à améliorer la santé, comprenant ledit extrait et/ou ladite fraction selon la revendication 1, comme principe actif unique ou en combinaison, utilisable dans le traitement de l'ulcère.
